(19) Europäisches Patentamt
European Patent Office
Office européen des brevets

(11) Veröffentlichungsnummer: **0 004 316**
**B1**

# (12) EUROPÄISCHE PATENTSCHRIFT

(45) Veröffentlichungstag der Patentschrift: **23.09.81**

(21) Anmeldenummer: **79100697.6**

(22) Anmeldetag: **08.03.79**

(51) Int. Cl.³: **C 07 C 121/75,** C 07 C 121/66, A 01 N 53/00, C 07 C 120/00, C 07 D 317/64, C 07 C 49/385, C 07 C 69/74

## (54) Cyclopropankarbonsäureester, Verfahren zu ihrer Herstellung und ihre Verwendung in der Schädlingsbekämpfung.

(30) Priorität: **16.03.78 CH 2868/78**
**15.02.79 CH 1483/79**

(43) Veröffentlichungstag der Anmeldung:
**03.10.79 Patentblatt 79/20**

(45) Bekanntmachung des Hinweises auf die Patenterteilung:
**23.09.81 Patentblatt 81/38**

(84) Benannte Vertragsstaaten:
**BE CH DE FR GB IT NL**

(56) Entgegenhaltungen:
**CHEMICAL ABSTRACTS, Vol. 65, 1966 Columbus, Ohio, USA, J. ARCT et al.: »The fungistatic activity of some aryloxycyclopropanecarboxylic acids and their hydroxyamide derivatives«, Zusammenfassung Nr. 11264h–11265a**
**BULLETIN DE LA SOCIETE CHIMIQUE DE FRANCE, 1966, Seiten 734–742, Paris FR**

(73) Patentinhaber: **CIBA-GEIGY AG, Patentabteilung Postfach, CH-4002 Basel (CH)**

(72) Erfinder: **Bissig, Peter, Dr., Alemannenstrasse 5, CH-4106 Therwil (CH)**
Erfinder: **Greuter, Hans, Dr., 75, Cos Cob Avenue, Cos Cob Connecticut 06807 (US)**
Erfinder: **Gsell, Laurenz, Dr., Maiengasse 56, CH-4056 Basel (CH)**

**EP 0 004 316 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Cyclopropankarbonsäureester, Verfahren zu ihrer Herstellung und ihre Verwendung in der Schädlingsbekämpfung

Die vorliegende Erfindung betrifft Cyclopropankarbonsäureester, Verfahren zu ihrer Herstellung und ihre Verwendung in der Schädlingsbekämpfung.

Die Cyclopropancarbonsäureester haben die Formel

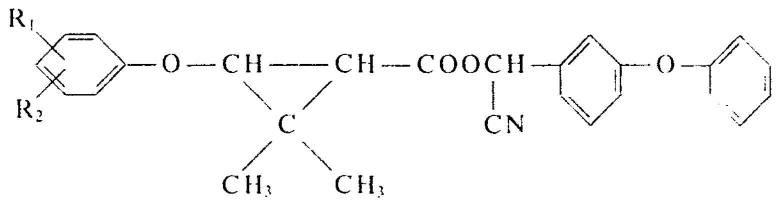

(I)

worin $R_1$ und $R_2$ je Wasserstoff, Halogen, Methyl, Methoxy, Trifluormethyl oder Cyano oder $R_1$ und $R_2$ zusammen Methylendioxy oder das Fragment $-CH=CH-CH=CH-$ bedeuten.

Unter Halogen sind Fluor, Chlor, Brom oder Jod insbesondere aber Fluor und Chlor, zu verstehen.

Die Verbindungen der Formel I können nach an sich bekannten Methoden z. B. wie folgt hergestellt werden:

1) 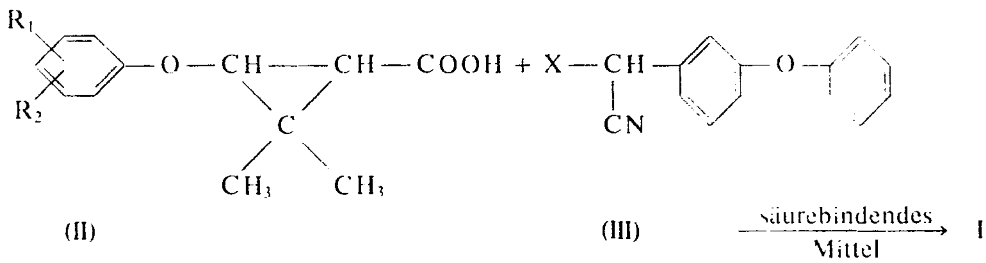


(II) (III) I

2) 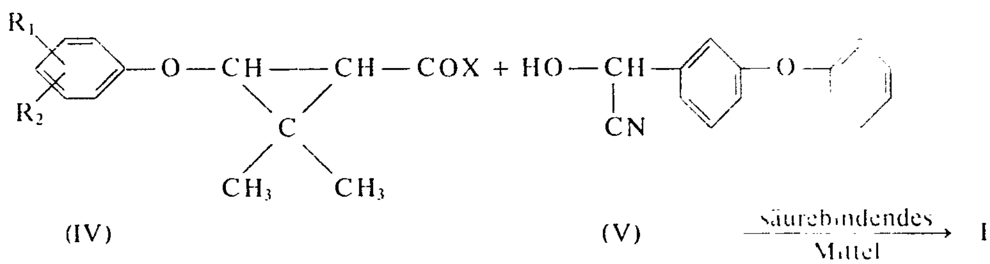


(IV) (V) I

3) 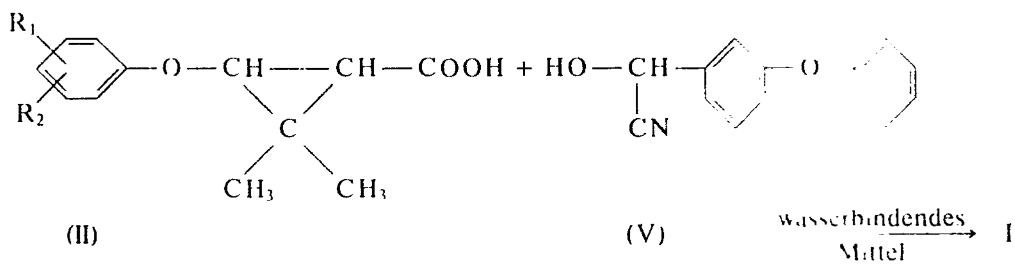


(II) (V) I

4) 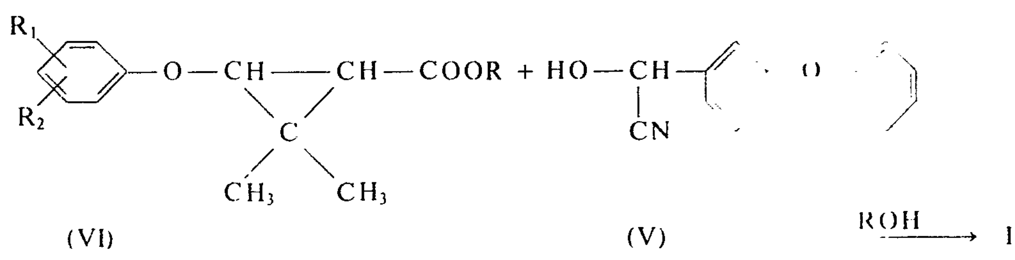


(VI) (V) I

In den Formeln II, IV und VI haben $R_1$ und $R_2$ die für die Formel I angegebene Bedeutung.

In den Formeln III und IV steht X für ein Halogenatom, insbesondere Chlor oder Brom und in der Formel VI steht R für $C_1-C_4$-Alkyl, insbesondere für Methyl oder Äthyl. Als säurebindendes Mittel für

die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z. B. Kalium-t.butylat und Natriummethylat in Betracht. Als wasserbindendes Mittel für das Verfahren 3 kann z. B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck vorzugsweise in einem inerten Lösungs- oder Verdünnungsmittel durchgeführt. Als Lösungs- oder Verdünnungsmittel eignen sich z. B. Äther und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis VI sind teilweise bekannt oder sie können analog bekannten Methoden hergestellt werden.

Die Verbindungen der Formel I können ebenfalls hergestellt werden, indem man die Verbindung der Formel

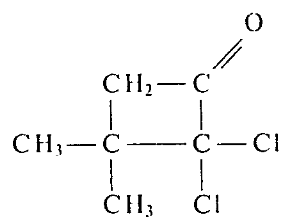

(VII)

in Gegenwart eines Katalysators in die Verbindung der Formel

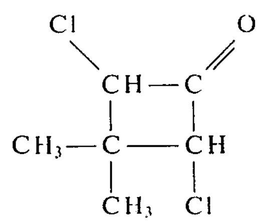

(VIII)

umlagert und die Verbindung der Formel VIII mit Phenolen der Formel

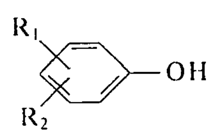

(IX)

worin $R_1$ und $R_2$ die für die Formel I angegebene Bedeutung haben, in Gegenwart einer Base in die Verbindung der Formel

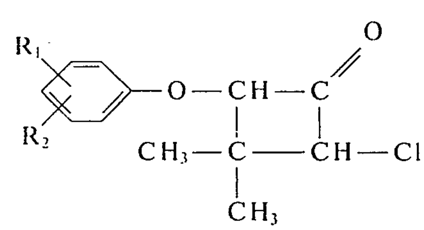

(X)

überführt, und anschließend mit einer Base die Ringverengung zu Verbindungen der Formel

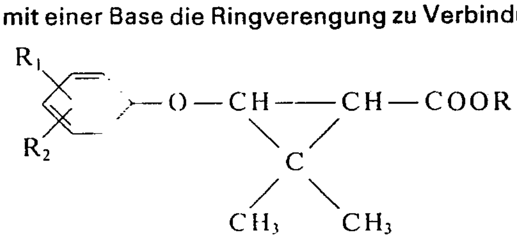

(XI)

durchführt, wobei R=H oder Niederalkyl bedeutet und die Verbindungen der Formel XI mit dem

Alkohol der Formel

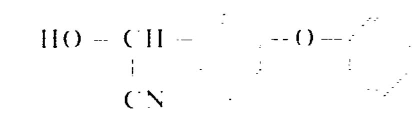

(V)

umsetzt.

In gewissen Fällen kann es vorteilhaft sein, wenn die Verbindung der Formel VII direkt mit Phenolen der Formel IX in Gegenwart einer Base zu den Verbindungen der Formel X umgesetzt werden. Die Verbindungen der Formel X kann man unter den bekannten Bedingungen der Favorski-Reaktion (Organic Reactions II, 261) in Gegenwart von R'OH in die Verbindungen der Formel II (R' = H) resp. der Formel VI R' = Alkyl) überführen. Die Verbindungen der Formel VII, VIII und X sind neu. Die Ausgangsverbindung der Formel VII kann auf an sich bekannte Weise aus Dichlorketen und Isobutylen dargestellt werden.

Verfahrensstufe 1

Für die Umlagerung der Verbindung der Formel VII in die Verbindung der Formel VIII können als Katalysatoren Säuren, Basen oder quaternäre Ammoniumhalogenide eingesetzt werden.

Als basische Katalysatoren kommen insbesondere organische Basen in Betracht, wie tertiäre Amine der Formel

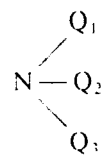

worin $Q_1$ Alkyl mit 1—8 C-Atomen, Cycloalkyl mit 5 oder 6 C-Atomen, Benzyl oder Phenyl und $Q_2$ und $Q_3$ unabhängig voneinander Alkyl mit 1—8 C-Atomen bedeuten, beispielsweise Triäthylamin, Tri-n-butylamin, Triisopentylamin, Tri n-octylamin, N,N-Dimethyl-cyclohexylamin, N,N-Dimethyl-benzylamin, N,N-Dimethyl-2-äthylhexylamin, N,N-Diäthylanilin; ferner cyclische Amine, wie Pyridin, Chinolin, Lutidin, N-Alkylmorpholine, wie N-Methylmorpholin, N-Alkylpiperidine, wie N-Methyl- und N-Äthylpiperidin, N-Alkylpyrrolidine, wie N-Methyl- und N-Äthylpyrrolidin; Diamine, wie N,N,N',N'-Tetramethyläthylendiamin, N,N,N',N'-Tetramethyl-1,3-diamino-butan, N,N'-Dialkylpiperazine, wie N,N'-Dimethylpiperazin; bicyclische Diamine, wie 1,4-Diazabicyclo[2.2.2]octan und bicyclische Amidine, wie 1,5-Diazabicyclo[5.4.0]undec-5-en und 1,5-Diazabicyclo[4.3.0]non-5-en, und schließlich polymere basische Verbindungen, wie p-Dimethylaminomethylpolystyrol.

Als saure Katalysatoren können z. B. anorganische oder organische Protonsäuren verwendet werden. Beispiele geeigneter anorganischer Protonsäuren sind Halogenwasserstoffsäuren, wie HCl, HBr, HF und HJ, Salpetersäure, Phosphorsäure und Schwefelsäure.

Als organische Protonsäuren kommen z. B. in Betracht: Sulfinsäuren, wie Benzolsulfinsäure; aliphatische und gegebenenfalls substituierte aromatische Sulfonsäuren, wie Methansulfonsäure, Benzolsulfonsäure, p-Toluolsulfonsäure, Naphthalinsulfonsäure, Naphthalin-1,5-disulfonsäuren; aliphatische Monocarbonsäuren mit vorzugsweise 1—18 C-Atomen wie Ameisensäure, Essigsäure, Propionsäure, Buttersäure, Laurinsäure, Palmitinsäure, Stearinsäure, halogenhaltige Monocarbonsäure, wie Chloressigsäure, Dichloressigsäure, Trichloressigsäure und Trifluoressigsäure; aliphatische Dicarbonsäuren mit vorzugsweise 2—12 Kohlenstoffatomen, wie Malonsäure, Bernsteinsäure, Adipinsäure, Sebacinsäure; gegebenenfalls substituierte aromatische Mono- oder Dicarbonsäuren, wie Benzoesäure, Toluylsäure, Naphthoesäure, Phthalsäure und Terphthalsäure; aliphatische und aromatische Phosphon- und Phosphinsäuren wie Methyl-, Benzyl- oder Phenylphosphonsäure oder Dimethyl- und Diäthylphosphonsäure oder Diäthyl- und Benzolphosphinsäure.

Werden Säuren oder Basen im Überschuß eingesetzt, so können sie auch als Lösungsmittel dienen.

Ferner können Salze von Protonensäuren, besonders Halogenwasserstoffsäuren, mit Ammoniak oder einer Stickstoff enthaltenden organischen Base sowie quaternäre Ammoniumhalogenide eingesetzt werden. Als Stickstoff enthaltende organische Basen eignen sich aliphatische, cycloaliphatische, araliphatische und aromatische primäre, sekundäre und tertiäre Amine sowie heterocyclische Stickstoffbasen. Als Beispiele seien genannt: primäre aliphatische Amine mit bis zu 12 C-Atomen, wie Methylamin, Äthylamin, n-Butylamin, n-Octylamin, n-Dodecylamin, Hexamethylendiamin, Dimethylamin, diäthylamin, Di-n-propylamin; tertiäre aliphatische Amine mit je 1—4 C-Atomen, in den Alkylteilen, wie Triäthylamin und Tri-n-butylamin; Cyclohexylamin, Dicyclohexylamin;

Benzylamin; gegebenenfalls substituierte primäre sekundäre und tertiäre aromatische Amine, wie Anilin, Toluidin, Naphthylamin, N-Methylanilin, Diphenylamin und N,N-Diäthylanilin; Pyrrolidin, Piperidin, N-Methyl-2-pyrrolidin, Piperazin, Pyridin, Picolin, Indolin, Chinuclidin, Morpholin, N-Methylmorpholin, 1,4-Diazabicyclo[2.2.2]octan.

Bevorzugt sind Salze der Formel

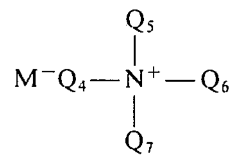

worin M Fluor, Brom oder Jod, insbesondere Chlor, $Q_4$ Wasserstoff, Alkyl mit 1—18 C-Atomen, Cyclohexyl, Benzyl, Phenyl oder Naphthyl und $Q_5$, $Q_6$ und $Q_7$ unabhängig voneinander Wasserstoff oder Alkyl mit 1—18 C-Atomen bedeuten, sowie N-Alkyl-Pyridiniumhalogenide mit 1—18 C-Atomen im Alkyl, besonders die entsprechenden Chloride.

Beispiele derartiger Salze sind: Ammoniumchlorid, Ammoniumbromid, Methylaminhydrochlorid, Cyclohexylaminhydrochlorid, Anilinhydrochlorid, Dimethylaminhydrochlorid, Di-isobutylaminohydrochlorid, Triäthylaminhydrochlorid, Triäthylaminhydrobromid, Tri-n-octylaminhydrochlorid, Benzyldimethylaminhydrochlorid, Tetramethyl-, Tetraäthyl-, Tetra-n-propyl-, Tetra-n-butylammoniumchlorid, -bromid und -jodid, Trimethyl-hexadecylammoniumchlorid, Benzyldimethylhexadecylammoniumchlorid, Benzyldimethyltetradecylammoniumchlorid, Benzyl-trimethyl-, -triäthyl- und tri-n-butylammoniumchlorid, n-Butyl-tri-n-propylammoniumbromid, Octadecyltrimethylammoniumbromid, Phenyltrimethylammoniumbromid oder -chlorid, Hexadecylpyridiniumbromid und -chlorid.

Als zusätzliche Co-Katalysatoren kann man Alkalimetallhalogenide, wie Kaliumjodid, Natriumjodid, Lithiumjodid, Kaliumbromid, Natriumbromid, Lithiumbromid, Kaliumchlorid, Natriumchlorid, Lithiumchlorid, Kaliumfluorid, Natriumfluorid und Lithiumfluorid, verwenden.

Die Menge des eingesetzten Katalysators kann innerhalb breiter Grenzen variieren. In manchen Fällen genügt es, wenn der Katalysator in Spuren vorliegt. Im allgemeinen wird jedoch der Katalysator bevorzugt in einer Menge von etwa 0,1 bis 15 Gewichtsprozent bezogen auf die Verbindung der Formel VII, eingesetzt.

Die Umlagerung kann sowohl in der Schmelze als auch in einem inerten organischen Lösungsmittel vorgenommen werden. Die Reaktionstemperaturen für die Umlagerung in der Schmelze liegen im allgemeinen etwa zwischen 60 und 150° C, insbesondere etwa 80 und 130° C.

Für die Umlagerung in der Schmelze eignen sich als Katalysatoren vor allem die vorerwähnten organischen Basen, insbesondere Trialkylamine mit je 1—8 C-Atomen in den Alkylteilen; ferner Salze von Halogenwasserstoffsäuren mit Ammoniak oder organischen stickstoffhaltigen Basen, wie Trialkylaminhydrochloride und -bromide mit je 1—8 C-Atomen in den Alkylteilen, und ganz besonders Tetraalkylammoniumhalogenide, vor allem -chloride, -bromide und -jodide, mit je 1—18 C-Atomen in den Alkylteilen.

Geeignete inerte organische Lösungsmittel sind z. B. gegebenenfalls nitrierte oder halogeniertealiphatische, cycloaliphatische oder aromatische Kohlenwasserstoffe, wie n-Hexan, n-Pentan, Cyclohexan, Benzol, Toluol, Xylole, Nitrobenzol, Chloroform, Tetrachlorkohlenstoff, Trichloräthylen, 1,1,2,2-Tetrachloräthan, Nitromethan, Chlorbenzol, Dichlorbenzole und Trichlorbenzole; niedere aliphatische Alkohole, z. B. solche mit bis zu 6 C-Atomen, wie Methanol, Äthanol, Propanol, Isopropanol, Butanole und Pentanole; aliphatische Diole, wie Äthylenglykol und Diäthylenglykol; Äthylenglykolmonoalkyläther und Diäthylenglykolmonoalkyläther mit je 1—4 C-Atomen in den Alkylteilen, wie Äthylenglykolmonomethyl- und -monoäthyläther, Diäthylenglykolmonomethyl- und -monoäthyläther; cyclische Amide wie N-Methyl-2-pyrrolidon, N-Acetyl-2-pyrrolidon und N-Methyl-ε-caprolactam; Amide der Kohlensäure, wie Tetramethylharnstoff und Dimorpholinocarbonyl; Amide der phosphorigen Säure, der Phosphorsäure, der Phenylphosphonsäure oder von aliphatischen Phosphonsäuren mit 1—3 C-Atomen im Säureteil, wie Phosphorsäuretriamid, Phosphorsäuretris(dimethylamid), Phosphorsäuretrimorpholid, Phosphorsäuretripyrrolinid, Phosphorsäure-bis(dimethylamid)-morpholid, Phosphorsäure-dimethylamid-diäthylamid-morpholid, Phosphorigsäure-tris(dimethylamid), Tetramethyldiamid der Methanphosphonsäure; Amide der Schwefelsäure, von aliphatischen oder aromatischen Sulfonsäuren, wie Tetramethylsulfid, Dimethylamid der Methansulfonsäure oder p-Toluolsulfonsäureamid; schwefelhaltige Lösungsmittel, wie organische Sulfone und Sulfoxide, z. B. Dimethylsulfoxid und Sulfolan; aliphatische und aromatische Nitrile, 3-Alkoxypropionitrile, aliphatische Ketone, Alkyl- und Alkoxyalkylester von aliphatischen Monocarbonsäuren, cyclische Äther, Dialkyläther, N,N-disubstituierte Amide von aliphatischen Monocarbonsäuren und Äthylenglykol- und Diäthylenglykoldialkyläther der unter der Verfahrensstufe 1) erwähnten Art.

Für die Umlagerung in Gegenwart eines sauren Katalysators verwendet man mit Vorteil polare Lösungsmittel, insbesondere niedere Alkohole, wie Methanol, Äthanol und Butanole, N,N-Dialkylamide von aliphatischen Monocarbonsäuren mit 1—3 C-Atomen im Säureteil, besonders N,N-Dimethylformamid, oder Dialkylsulfoxide, wie Dimethylsulfoxid,

In aprotischen, stark polaren Lösungsmitteln, wie den vorerwähnten N,N-disubstituierten Amiden von aliphatischen Monocarbonsäuren, cyclischen Amiden, Amiden der Kohlensäure, Amiden der phosphorigen Säure, der Phosphorsäure, der Phenylphosphonsäure oder von aliphatischen Phosphonsäuren, Amiden der Schwefelsäure oder von aliphatischen oder aromatischen Sulfonsäuren, sowie Dialkylsulfoxiden, wie Dimethylsulfoxid, läuft die Reaktion auch ohne Zusatz von Base oder Säure ab. In diesen Fällen wirkt das Lösungsmittel als Katalysator.

Im allgemeinen wird jedoch bei der Umlagerung in Gegenwart eines inerten organischen Lösungsmittels ein Katalysator zugesetzt, bevorzugt eine organische Base mit einem $pK_a$-Wert von über 9, insbesondere Trialkylamine mit je 1—8 C-Atomen in den Alkylteilen, wie Triäthylamin, Tri-n-butylamin und Tri-n-octylamin; ferner Halogenwasserstoffsäuren, besonders HCl und HBr, sowie Tetraalkylammoniumhalogenide, besonders -chloride, -bromide und -jodide, mit je 1—18 C-Atomen in den Alkylteilen.

Besonders bevorzugte Lösungsmittel sind aliphatische Alkohole mit 2—4 C-Atomen, Toluol, Xylole, Chlorbenzol, Dioxan, Acetonitril, 3-Methoxypropionitril, Äthylenglykoldiäthyläther und Di-isopropylketon.

Die Reaktionstemperaturen für die Umlagerung in Gegenwart eines inerten organischen Lösungsmittels liegen im allgemeinen zwischen etwa 0 und 150°C, bevorzugt zwischen etwa 80 und 130°C.

## Verfahrensstufe 2

Die Überführung der Dichlorcyclobutanone der Formel VII oder der Formel VIII in die Verbindungen der Formel X erfolgt definitionsgemäß mit einem Phenol der Formel IX in Gegenwart einer Base. Als solche kommen beispielsweise organische Basen wie tertiäre Amine, vor allem Trialkylamine mit je 1—4, insbesondere 2—4 Kohlenstoffatomen in den Alkylteilen; cyclische Amine, wie Pyridin, Chinolin, N-Alkyl-Pyrrolidine, N-Alkyl-Piperidine, N,N-Dialkyl-Piperazine und N-Alkyl-Morpholine oder Dialkylaniline mit je 1 oder 2 Kohlenstoffatomen in den Alkylteilen, wie N-Methylpyrrolidin, N-Äthylpiperidin, N,N'-Dimethylpiperazin, N-Äthylmorpholin und Dimethylanilin, sowie bicyclische Amidine, wie 1,5-Diazabicyclo[5.4.0]undec-5-en und 1,5-Diazabicyclo-[4.3.0]non-5-en, und bicyclische Diamine, wie 1,4-Diazabicyclo[4.3.0]non-5-en, und bicyclische Diamine, wie 1,4-Diazabicyclo[2.2.2]octan in Betracht.

Bevorzugte organische Basen sind Trialkylamine mit je 2—4 C-Atomen in den Alkylteilen, besonders Triäthylamin, und Pyridin. Die organische Base kann gleichzeitig auch als Lösungsmittel dienen.

Als organische Basen können Alkali- und Erdalkalihydroxide, Carbonate und Hydrogencarbonate, wie zum Beispiel Kalium-, Natrium-, Calcium- und Barium-hydroxid, Natrium- und Kaliumcarbonat sowie Natrium- und Kaliumhydrogencarbonat eingesetzt werden. Als besonders vorteilhaft erwiesen sich Natrium- und Kaliumhydroxid sowie Natrium- und Kaliumcarbonat.

Geeignete Lösungsmittel sind zum Beispiel gegebenenfalls halogenierte aromatische oder aliphatische Kohlenwasserstoffe, wie Benzol, Toluol, Xylole, Chlorbenzol, Dichlor- und Trichlorbenzole, n-Pentan, n-Hexan, n-Octan, Methylenchlorid, Chloroform, Tetrachlormethan, 1,1,2,2-Tetrachloräthan und Trichloräthylen; cycloaliphatische Kohlenwasserstoffe, wie Cyclopentan oder Cyclohexan; cycloaliphatische Ketone, wie Cyclopentanon und Cyclohexanon, sowie aliphatische Ketone, aliphatische und cyclische Äther, Alkylnitrile und 3-Alkoxypropionitrile.

Gute Ergebnisse, insbesondere mit Natrium- und Kaliumhydroxid als Basen, liefern auch 2phasige Lösungsmittelgemische bestehend aus Wasser und einem der obenstehend genannten Lösungsmittel, wie z. B. das System Wasser/Chloroform. Die Base wird in mindestens äquimolaren Mengen bezogen auf die Cyclobutanone der Formel VII oder VIII verwendet. Die Reaktionstemperaturen sind im allgemeinen nicht kritisch. Sie liegen im allgemeinen zwischen −20°C und 100°C, bevorzugt zwischen −10°C und 50°C.

## Verfahrensstufe 3

Die Überführung der Cyclobutanone der Formel X in die Verbindungen der Formel IV resp. VI unter Ringverengung erfolgt naturgemäß in Gegenwart einer Base. Als solche kommen beispielsweise Hydroxide bzw. Alkohole der Formel XI

$$M_I^{n+}(R'O^-)_n \qquad (XI)$$

in Betracht, worin $M_I$ ein Alkalimetall oder Erdalkalimetallkation und n die Zahl 1 oder 2 bedeuten und R' die unter Formel II resp. V angegebene Bedeutung hat, wie Natrium-, Kalium-, Calcium- und Bariumhydroxid oder Natrium- und Kaliummethylat, -äthylat, -isopropylat, -sek-butylat, -tert-butylat, Magnesiummethylat.

Geeignete Basen snd ferner Alkalimetall- und Erdalkalimetallcarbonate und Alkalimetallhydrogencarbonate, wie Calcium-, Barium-, Kalium- und Natriumcarbonat, Natrium- und Kaliumhydrogencarbonat.

Unter Umständen kann die Reaktion auch durch Zusatz von geeigneten Halogenidakzeptoren, beispielsweise Silbernitrat, beschleunigt werden.

Diese Basen werden in mindestens stöchiometrischer Menge, bevorzugt jedoch im Überschuß eingesetzt.

Die Umsetzung wird je nach Art der Base zweckmäßig in wäßrigem, wäßrig-organischem oder organischem Medium vorgenommen. Wird als Base ein Alkalimetall- oder Erdalkalimetallcarbonat verwendet, so wird die Umsetzung in wäßrigem oder wäßrig-organischem Medium durchgeführt. Auch die Umsetzung in Gegenwart von Alkalimetall- oder Erdalkalimetallhydroxiden und Alkalimetallhydrogencarbonaten wird mit Vorteil in wäßrigem oder wäßrig-organischem Medium vorgenommen. Dabei werden nach Ansäuern des Reaktionsgemisches, z. B. durch Zugabe von konzentrierter Salzsäure, Verbindungen der Formel II mit R'=H erhalten, die auf an sich bekannte Weise in Derivate der Formel II in IV übergeführt werden können.

Geeignete organische Lösungsmittel für die Umsetzung in wäßrig-organischem oder organischem Medium sind niedere Alkohole, z. B. solche mit bis zu 6 C-Atomen, wie Methanol, Äthanol, Propanol, Isopropanol, Butanol, sek-Butanol, tert-Butanol und Amylalkohole; aliphatische oder cyclische Äther, wie Diäthyläther, Di-n-propyläther, Di-isopropyläther, Tetrahydrofuran, Tetrahydropyran und Dioxan; sowie aliphatische, cycloaliphatische oder aromatische Kohlenwasserstoffe, wie n-Pentan, n-Hexan, Cyclohexan, Benzol, Toluol und Xylole.

Bevorzugt ist die Umsetzung in Gegenwart von Hydroxiden bzw. Alkoholaten der Formel XI, worin n die Zahl 1, $M_1$ ein Alkalimetallkation, besonders Natrium oder Kalium und R' Wasserstoff oder Alkyl mit 1—4 C-Atomen bedeuten. Ganz besonders bevorzugt ist die Umsetzung zu Verbindungen der Formel II in Gegenwart von NaOH oder KOH in wäßrigem oder wäßrig-organischem Medium, z. B. in wäßrigen Alkoholen, wie wäßrigem Methanol oder Äthanol, oder in wäßrigem Dioxan.

Die Umsetzung wird im allgemeinen beim Siedepunkt des gewählten Reaktionsmediums vorgenommen. Besonders geeignet sind Temperaturen zwischen etwa 40 und 120° C.

Die Zwischenprodukte der Formeln II resp. VI, VII und X sind neu. Diese Zwischenprodukte wie auch die Endprodukte der Formel I können auf an sich bekannte Weise isoliert und gereinigt werden. Eine Aufarbeitung der Zwischenprodukte ist jedoch nicht unbedingt erforderlich.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zwecken z. B. der Ordnungen Lepidoptera, Coleoptera, Homoptera, Heteroptera, Diptera, Acarina, Thysanoptera, Orthoptera, Anoplura, Siphoneptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Fraßinsekten, in Zier- und Nutzpflanzen, insbesondere in Baumwollkulturen (z. B. gegen Spodotera littoralis und Heliothis virescens) und Gemüsekulturen (z. B. gegen Leptinotarsa decemlineata und Myzus persicae).

Wirkstoffe der Formel I zeigen auch eine sehr günstige Wirkung gegen Fliegen wie z. B. Musca domestica und Mückenlarven.

Die akarizide bzw. insektizide Wirkung läßt sich durch Zusatz von anderen Insektiziden und/oder Akariziden wesentlich verbreitern und an gegebene Umstände anpassen. Als Zusätze eignen sich z. B. org. Phosphorverbindungen; Nitrophenole und deren Derivate; Formamidine; Harnstoffe; andere pyrethrinartige Verbindungen sowie Carbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u. a. Piperonlybutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Verbindungen der Formel I können für sich allein oder zusammen mit geeigneten Träger- und/oder Zuschlagstoffen eingesetzt werden. Geeignete Zuschlagstoffe können fest oder flüssig sein und entsprechen den in der Formulierungstechnik üblichen Stoffen wie z. B. natürlichen oder regenerierten Stoffen, Lösungs-, Dispergier-, Netz-, Haft-, Verdickungs-, Binde- und/oder Düngemitteln.

Die Herstellung erfindungsgemäßer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösunsmitteln.

Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;
b) Lösungen

Der Gehalt an Wirkstoff in den oben beschriebenen Mitteln liegt zwischen 0,1 bis 95%, dabei ist zu erwähnen, daß bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

Stäubemittel

Zur Herstellung eines a) 5%igen und b) 2%igen Stäubemittels werden die folgenden Stoffe verwendet:

a) 5 Teile Wirkstoff
95 Teile Talkum
b) 2 Teile Wirkstoff
1 Teil hochdisperse Kieselsäure
97 Teile Talkum.

Der Wirkstoff wird mit den Trägerstoffen vermischt und vermahlen.

Granulat

Zur Herstellung eines 5%igen Granulates werden die folgenden Stoffe verwendet:

5 Teile Wirkstoff
0,25 Teile Epichlorhydrin
0,25 Teile Cetylpolyglykoläther
3,50 Teile Polyäthylenglykol
91 Teile Kaolin (Korngröße 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschließend wird das Aceton im Vakuum verdampft.

Spritzpulver

Zur Herstellung eines a) 40%igen, b) und c) 25%igen, d) 10%igen Spritzpulvers werden folgende Bestandteile verwendet:

a) 40 Teile Wirkstoff
5 Teile Ligninsulfonsäure-Natriumsalz
1 Teil Dibutylnaphthalinsulfonsäure-Natriumsalz
54 Teile Kieselsäure;
b) 25 Teile Wirkstoff
4,5 Teile Calcium-Ligninsulfonat
1,9 Teile Champagne-Kreide/Hydroxyäthylcellulose-Gemisch (1 : 1)
1,5 Teile Natrium-dibutyl-naphthalinsulfonat
19,5 Teile Kieselsäure
19,5 Teile Champagne-Kreide
28,1 Teile Kaolin;
c) 25 Teile Wirkstoffe
2,5 Teile Isooctylphenoxy-polyäthylen-äthanol

1,7 Teile Champagne-Kreide/Hydroxyäthylcellulose-Gemisch (1 : 1)
8,3 Teile Natriumaluminiumsilikat
16,5 Teile Kieselgur
46 Teile Kaolin;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

## Emulgierbare Konzentrate

Zur Herstellung eines a) 10%igen b) 25%igen und c) 50%igen emulgierbaren Konzentrates werden folgende Stoffe verwendet.

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylfomamid
43,2 Teile Xylol;

b) 25 Teile Wirkstoff
2,5 Teile epoxydiertes Pflanzenöl
10 Teile eines Alkylarylsulfonat/Fettalkoholpolyglykoläther-Gemisches
5 Teile Dimethylfomamid
57,5 Teile Xylol;

c) 50 Teile Wirkstoff
4,2 Teile Tributylphenol-Polyglykoläther
5,8 Teile Calcium-Dodecylbenzolsulfonat
20 Teile Cyclohexan
20 Teile Xylol.

Aus solchen Konzentraten können durch Verdünnen mit Wasser Emulsionen jeder gewünschten Konzentration hergestellt werden.

## Sprühmittel

Zur Herstellung eines a) 5%igen und b) 95%igen Sprühmittels werden die folgenden Bestandteile verwendet:

a) 5 Teile Wirkstoff
1 Teil Epichlorhydrin
94 Teile Benzin (Siedegrenzen 160—190° C);

b) 95 Teile Wirkstoff
5 Teile Epichlorhydrin.

## Beispiel 1

### Herstellung von
### 2,2-Dimethyl-3-(4-chlorphenoxy)-cyclopropankarbonsäure-3'-(phenoxy)-$\alpha$-cyanobenzylester.

4,14 g (0,016 Mol) 2,2-Dimethyl-3-(4-chlorphenoxy)-cyclopropankarbonsäure werden mit 4,2 ml Oxalylchlorid versetzt und 30 Minuten bei 25° C und 15 Minuten bei 50° C gerührt. Das Reaktionsgemisch wird eingeengt, zweimal mit je 3 ml Tetrachlorkohlenstoff versetzt und eingeengt. Das rohe Säurechlorid wird mit 4,25 g (0,016 Mol) 3-Phenoxy-$\alpha$-cyanobenzylalkohol in 8 ml Toluol vorgelegt und 4 ml Pyridin in 4 ml Toluol derart zugetropft, daß die Temperatur nicht über 0° C steigt. Das Reaktionsgemisch wird 2 Stunden bei Raumtemperatur ausgerührt, mit 0,5— ml Wasser versetzt, 15 Minuten nachgerührt und mit 6 N-Schwefelsäure sauer gestellt. Nach der Extraktion mit Äther, dem Waschen mit Bikarbonatlösung, dem Trocknen mit Natriumsulfat und dem Einengen wird das

Rohprodukt an Kieselgel mit Hexan/Äther (Volumenverhältnis 10 : 1) als Eluiermittel chromatographiert.

Man erhält die Verbindung der Formel

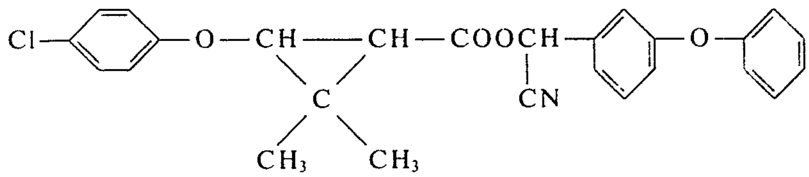

als Öl mit einem Brechungsindex von $n_D^{20°} = 1,5732$

Auf analoge Weise werden auch folgende Verbindungen hergestellt:

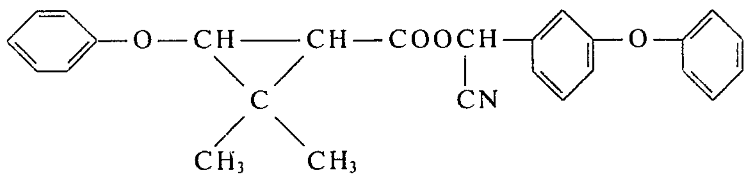

$n_D^{20°} = 1,5680$

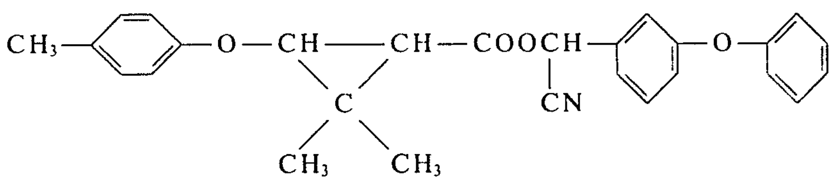

$n_D^{20°} = 1,5643$

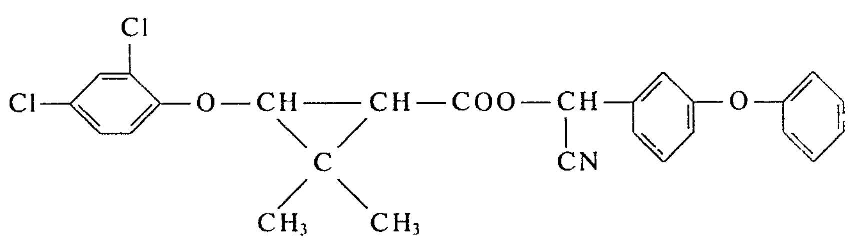

$n_D^{20°} = 1,5769$

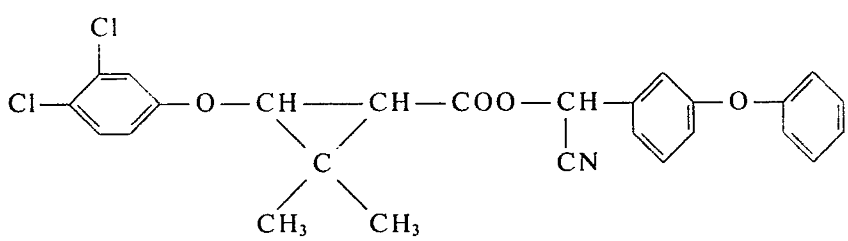

$n_D^{20°}$ 1,5780

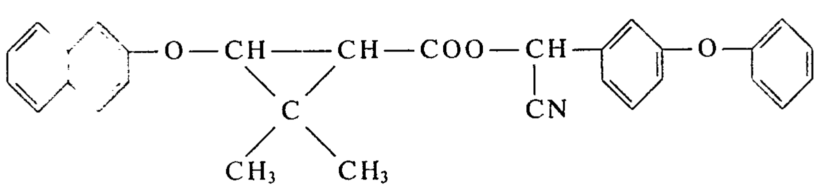

$n_D^{20°} = 1,5936$

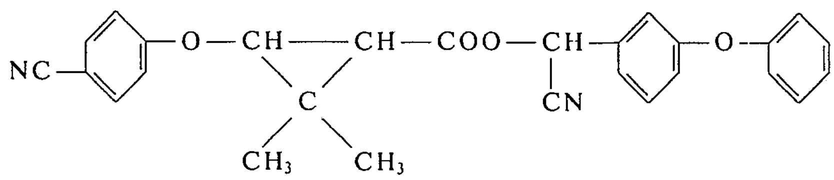

Diastereomere, Smp. 86–88°C bzw. 137–138°C

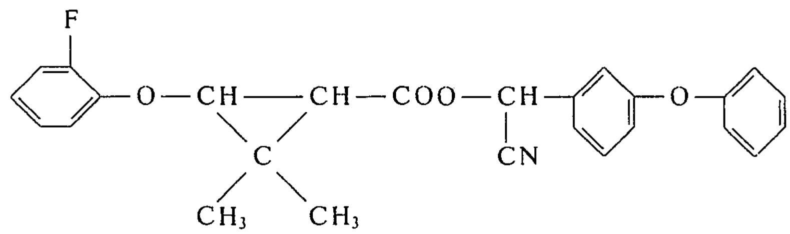

$n_D^{20°} = 1.5553$

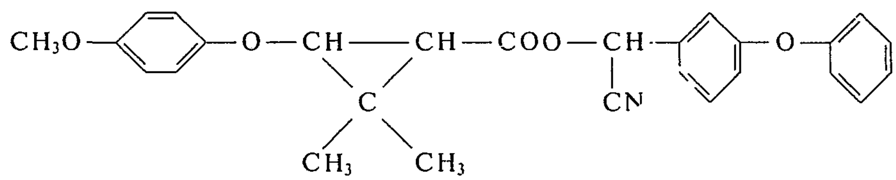

$n_D^{20°} = 1,5631$

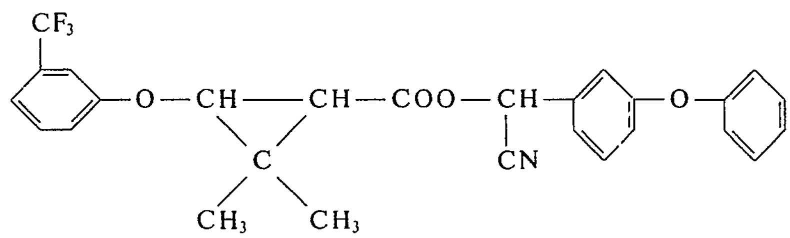

$n_D^{20°} = 1,5293$

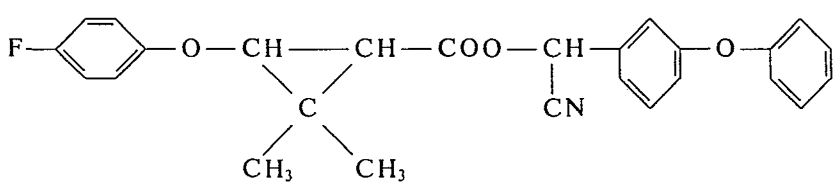

$n_D^{20°} = 1,5498$

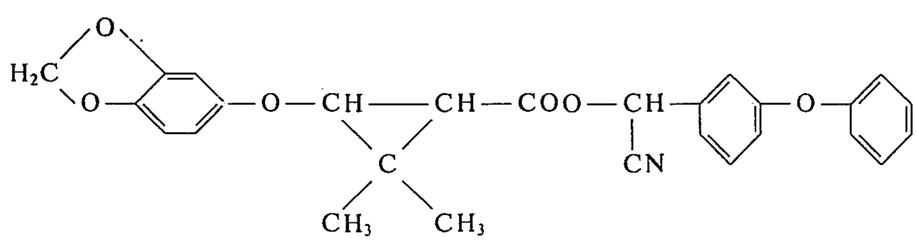

$n_D^{20°} = 1,5745$

### Herstellung von 2,2-Dichlor-3,3-dimethyl cyclobutanon

In einem 6,3 l Autoklaven werden 295 g (2,0 Mol) Dichloracetylchlorid und 800 ml Pentan vorgelegt. Danach wird der Autoklav verschlossen und bei Raumtemperatur 1120 g (20 mol) Isobutylen eingepreßt und anschließend auf 70°C geheizt. Eine Lösung bestehend aus 202 g (2,0 Mol) Triäthylamin und 800 ml Pentan werden dann während 4 Stunden eingepreßt und das

Reaktionsgemisch noch weitere 4 Stunden bei 70°C gerührt (Maximaldruck 9 bar). Nach erfolgtem Abkühlen wird abgenutscht, dreimal mit je 300 ml Pentan nachgewaschen und die Mutterlauge destilliert; vorerst bei Normaldruck das Pentan entfernt und hernach bei 73—74°/11 mm die Verbindung der Formel

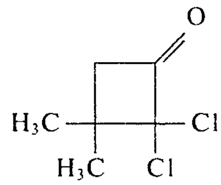

destilliert, welche im Kühlschrank kristallisiert.

2,4-Dichlor-3,3-dimethylcyclobutanon

501 g (3 Mol) 2,2-Dichlor-3,3-dimethylcyclobutanon, werden in 200 ml Toluol zum Rückfluß erhitzt. Dazu werden über 2,5 Std. 15,2 g Triäthylamin in 30 ml Toluol zugegeben. Das Reaktionsgemisch wird über Nacht bei Raumtemperatur ausgerührt, klarfiltriert und eingedampft. Der Rückstand wird unter vermindertem Druck destilliert. Man erhält die Verbindung der Formel

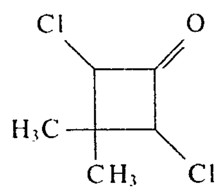

mit Siedepunkt 85°C/14 mm.

Herstellung von 2-(p-Chlorphenoxy)-3,3-dimethyl-4-chlor cyclobutanon aus 2,2-Dichlor-3,3-dimethyl cyclobutanon

41,8 g (0,25 Mol) 2,2-Dichlor-3,3-dimethyl cyclobutanon, 32,1 g (0,25 Mol) p-Chlorphenol und 5,0 g Tetrabutylammoniumchlorid werden in 100 ml Chloroform vorgelegt. Die hellbraune Lösung wird auf 5°C gekühlt und dann bei 5—9°C während 55 Minuten 100 ml 10prozentige wäßrige Natronlauge zugetropft. Anschließend wird bei dieser Temperatur während 15 Minuten weitergerührt. Dann wird viermal mit je 75 ml Äther extrahiert, die vereinigten Extrakte mit 100 ml Sole gewaschen, über Natriumsulfat getrocknet und eingedampft. Das Rohprodukt wird an Kieselgel mit Toluol/Hexan (Volumenverhältnis 1 : 1) als Eluiermittel chromatographiert. Man erhält die Verbindung der Formel

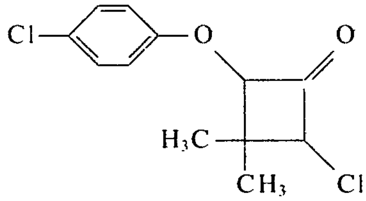

als weiße Kristalle vom Smp. 72—75°C.

Herstellung von 2(p-Methylphenoxy)3,3-dimethyl-4-chlor cyclobutanon aus 2,4-Dichlor-3,3-dimethyl cyclobutanon

16,7 g (0,1 Mol) 2,4-Dichlor-3,3-dimethyl cyclobutanon werden in 30 ml Chloroform vorgelegt, auf ca. 5°C gekühlt und bei dieser Temperatur 10,1 g (105 mMol) p-Kresol und eine Lösung bestehend aus 2,0 g Tetrabutylammoniumchlorid und 10 ml Chloroform zugegeben. Während 45 Minuten werden dann 40 ml 10prozentige wäßrige Natronlauge bei ca. 5°C zugetropft und anschließend weitere 45 Minuten bei Raumtemperatur gerührt. Das Reaktionsgemisch wird zweimal mit je 60 ml Äther extrahiert und die vereinigten organischen Phasen mit Sole gewaschen. Nach dem Trocknen mit

Natriumsulfat wird eingedampft und das Rohprodukt an Kieselgel chromatographiert (Petroläther/Toluol 4 : 1). Man erhält die Verbindung der Formel

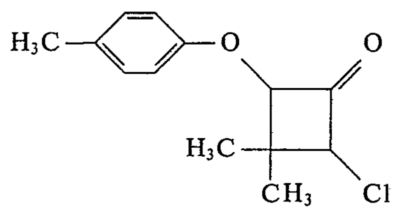

als Kristalle vom Smp 61—61,5° C.

Herstellung von 2,2-Dimethyl-3-(p-methylphenoxy)-cyclopropankarbonsäure

40 ml 10prozentige wäßrige Natronlauge werden auf ca. −8° C gekühlt. Dazu wird bei −5 bis −10° C eine Lösung bestehend aus 7,16 g (30 mMol) 2-Chlor-3,3-dimethyl-4-(p-methylphenoxy)-cyclobutanon und 16 ml Dioxan während 35 Minuten unter Rühren zugetropft. Anschließend wird eine weitere halbe Stunde bei −5° C gerührt und dann bei Raumtemperatur über Nacht stehen gelassen. Zur leicht trüben gelben Lösung werden 30 ml Äther gegeben und nach kurzem Rühren die Phasen getrennt. Die wäßrige Phase wird mit 6 N Schwefelsäure angesäuert und dreimal mit je 30 ml Äther extrahiert. Die Äther-Extrakte werden mit Sole gewaschen, mit Natriumsulfat getrocknet und eingedampft. Man erhält die Verbindung der Formel

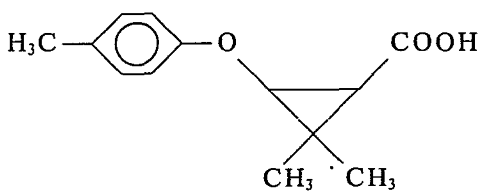

als weiße Kristalle vom Fp = 122—124° C.

Beispiel 2

A) Insektizide Fraßgift-Wirkung

Baumwollpflanzen wurden mit einer 0,05%igen wäßrigen Wirkstoffemulsion (erhalten aus einem 10%igen emulgierbaren Konzentrat) besprüht. Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt. Verbindungen gemäß Beispiel 1 zeigten im obigen Test eine gute insektizide Fraßgift-Wirkung gegen Spodoptera- und Heliothis-Larven.

Beispiel 3

Akarizide Wirkung

Phaseolus vulgaris Pflanzen wurden 12 Stunden vor dem Test auf akarizide Wirkung mit einem infestierten Blattstück aus einer Massezucht von Tetranychus urticae belegt. Die übergelaufenen beweglichen Stadien wurden aus einem Chromatographiezerstäuber mit den emulgierten Testpräparaten derart besprüht, daß kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der »Haltezeit« standen die behandelten Pflanzen in Gewächshauskabinen bei 25° C. Verbindungen gemäß Beispiel 1 wirkten im obigen Test gegen Adulte, Larven und Eier von Tetranychus urticae.

Beispiel 4

Wirkung gegen Zecken

A) Thipicephalus bursa

Je 5 adulte Zecken bzw. 50 Zeckenlarven wurden in ein Glasröhrchen gezählt und für 1 bis 2 Minuten in 2 ml einer wäßrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte. Die Auswertung erfolgte bei den Adulten nach 2 Wochen und bei den Larven nach 2 Tagen. Für jeden Versuch liefen 2 Wiederholungen.

B) Boophilus microplus Larven)

Mit einer analogen Verdünnungsreihe wie beim Test A) wurden mit je 20 sensiblen resp. OP-resistenten Larven Versuche durchgeführt. (Die Resistenz bezieht sich auf die Verträglichkeit von Diazinon). Verbindungen gemäß Beispiel 1 wirkten in diesen Tests gegen Adulte und Larven von Rhipicephalus bursa und sensible resp. OP-resistente Larven von Boophilus microplus.

**Patentansprüche**

1. Ein Cyclopropankarbonsäureester der Formel

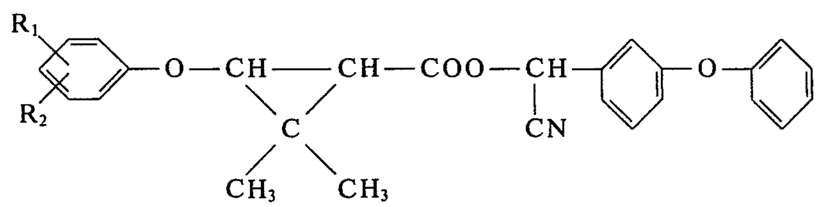

worin $R_1$ und $R_2$ je Wasserstoff, Halogen, Methyl, Methoxy, Trifluormethyl oder Cyano oder $R_1$ und $R_2$ zusammen Methylendioxy oder das Fragment $-CH=CH-CH=CH-$ bedeuten.

2. Die Verbindung gemäß Anspruch 1 der Formel

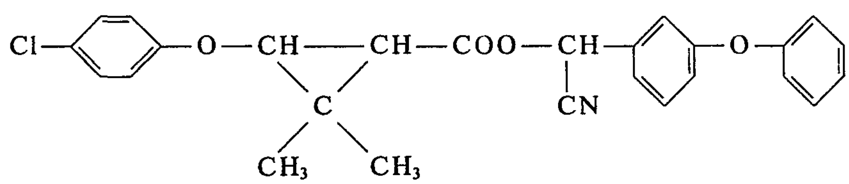

3. Die Verbindung gemäß Anspruch 1 der Formel

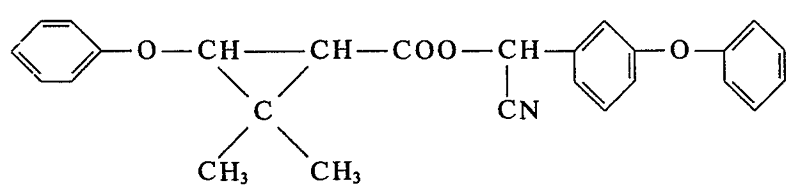

4. Die Verbindung gemäß Anspruch 1 der Formel

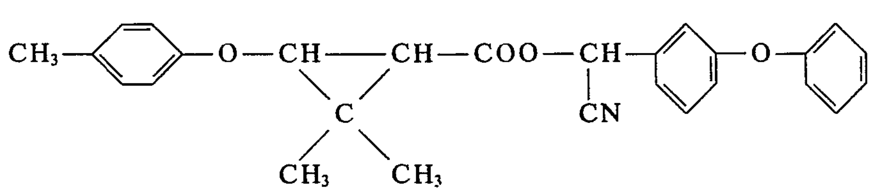

5. Die Verbindung gemäß Anspruch 1 der Formel

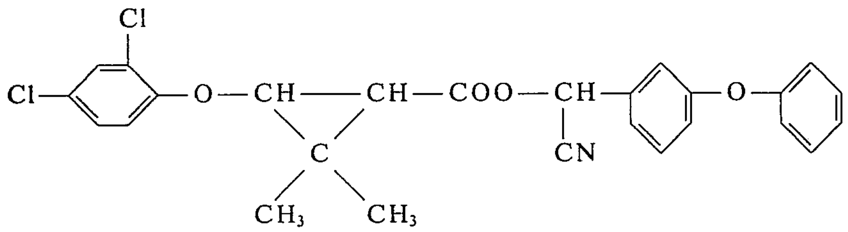

6. Die Verbindung gemäß Anspruch 1 der Formel

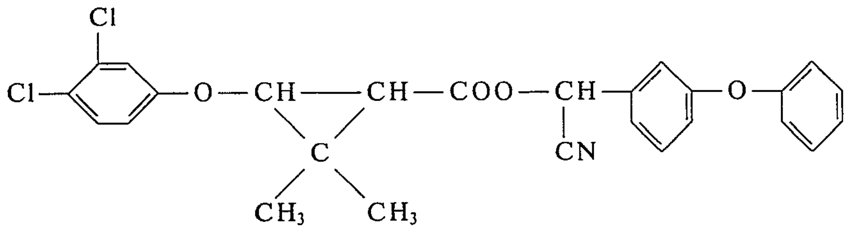

7. Die Verbindung gemäß Anspruch 1 der Formel

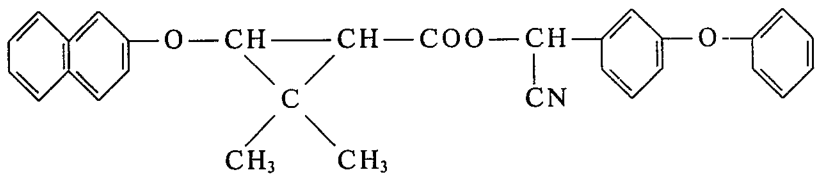

8. Die Verbindung gemäß Anspruch 1 der Formel

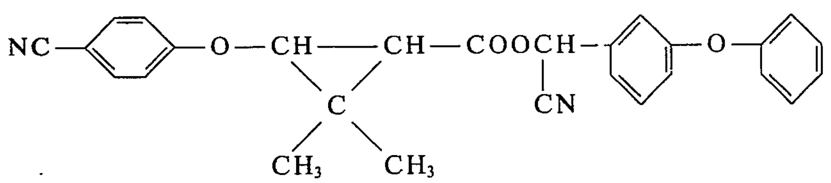

9. Die Verbindung gemäß Anspruch 1 der Formel

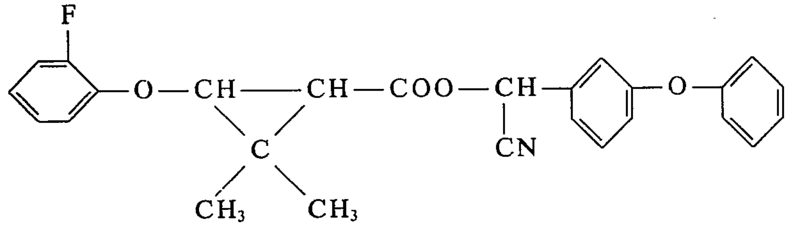

10. Die Verbindung gemäß Anspruch 1 der Formel

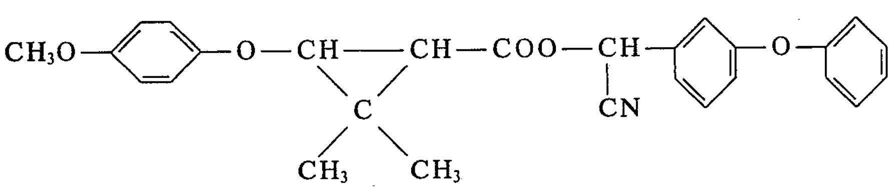

11. Die Verbindung gemäß Anspruch 1 der Formel

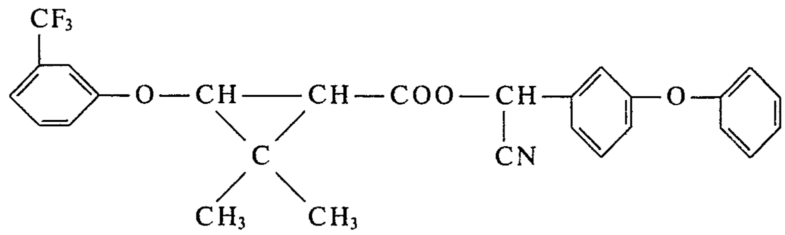

12. Die Verbindung gemäß Anspruch 1 der Formel

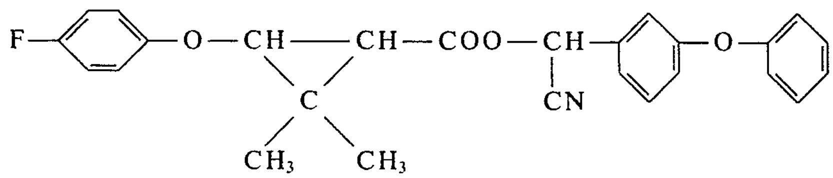

13. Die Verbindung gemäß Anspruch 1 der Formel

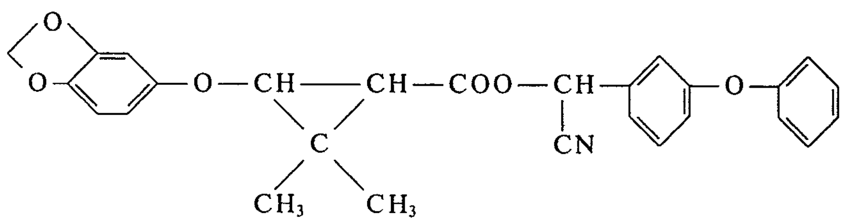

14. Verfahren zur Herstellung einer Verbindung gemäß Anspruch, dadurch gekennzeichnet, daß man eine Verbindung der Formel

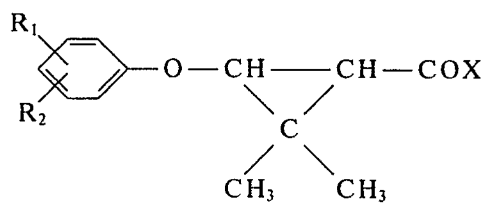

worin $R_1$ und $R_2$ die im Anspruch 1 angegebene Bedeutung haben und X für Halogen steht, in Gegenwart eines säurebindenden Mittels mit der Verbindung der Formel

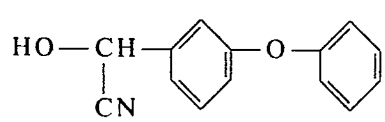

umsetzt.

15. Verfahren zur Herstellung einer Verbindung gemäß Anspruch 1, dadurch gekennzeichnet, daß man die Verbindung der Formel VII

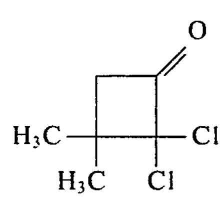

(VII)

in Gegenwart eines Katalysators in eine Verbindung der Formel VIII

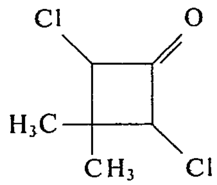

(VIII)

umlagert und die Verbindungen VII oder VIII mit Phenolen der Formel IX

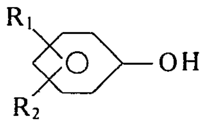

(IX)

worin $R_1$ und $R_2$ die in Anspruch 1 gegebene Bedeutung haben, in Verbindungen der Formel X

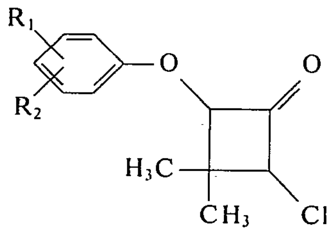

(X)

überführt, und anschließend definitionsgemäß mit einer Base die Ringverengung zu Verbindungen der Formel XI

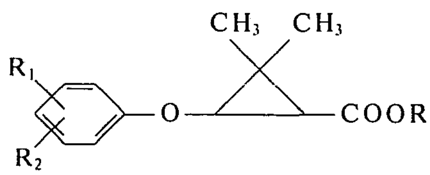

(XI)

durchführt, wobei R=H oder Niederalkyl bedeutet und die Verbindungen der Formel XI mit dem Alkohol der Formel

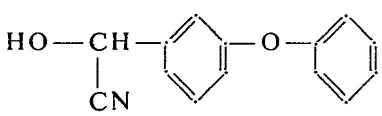

(V)

umsetzt.

16. Ein Schädlingsbekämpfungsmittel, welches als aktive Komponente eine Verbindung gemäß Anspruch 1 und einen geeigneten Träger- und/oder anderen Zuschlagstoff enthält.

17. Verwendung einer Verbindung gemäß Anspruch 1 zur Bekämpfung von verschiedenartigen tierischen und pflanzlichen Schädlingen.

18. Verwendung gemäß Anspruch 17 zur Bekämpfung von Insekten von Vertretern der Ordnung Akarina.

**Claims**

1. A cyclopropanecarboxylic acid ester of the formula

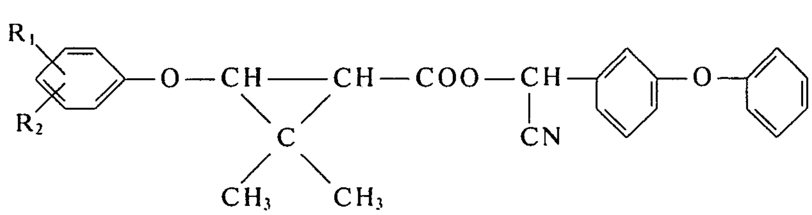

wherein each of $R_1$ and $R_2$ is hydrogen, halogen, methyl, methoxy, trifluormethyl or cyano, or $R_1$ and $R_2$ together are methylenedioxy or the fragment $-CH=CH-CH=CH-$.

2. The compound according to claim 1 of the formula

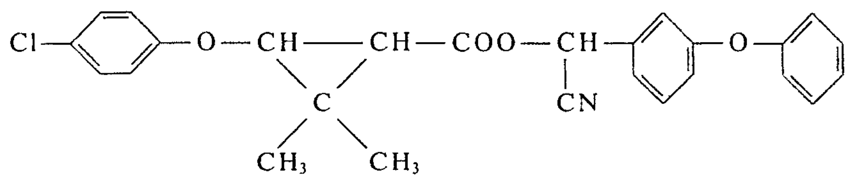

3. The compound according to claim 1 of the formula

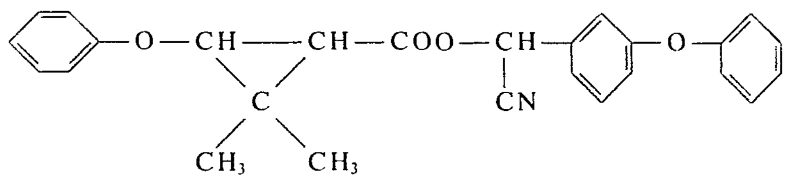

4. The compound according to claim 1 of the formula

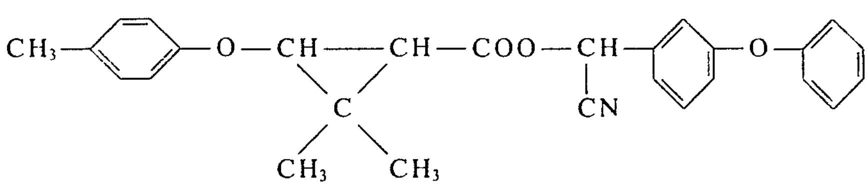

5. The compound according to claim 1 of the formula

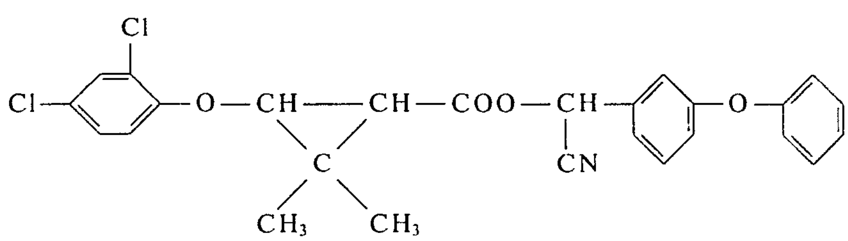

6. The compound according to claim 1 of the formula

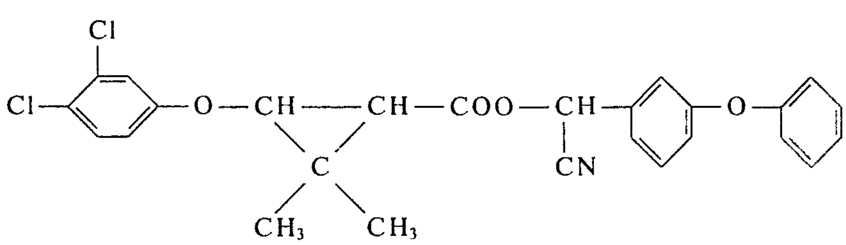

7. The compound according to claim 1 of the formula

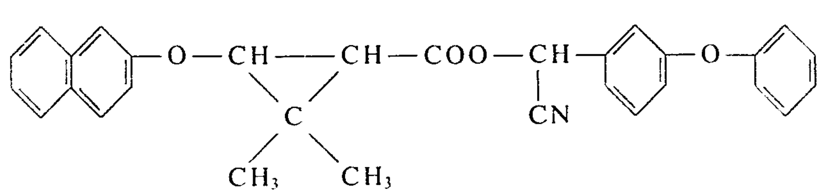

8. The compound according to claim 1 of the formula

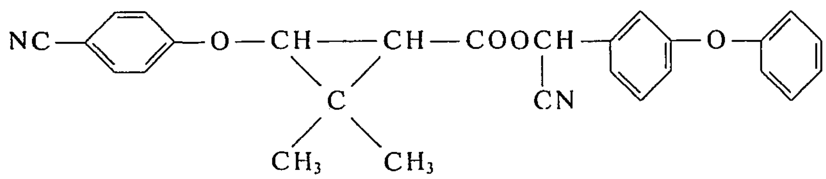

9. The compound according to claim 1 of the formula

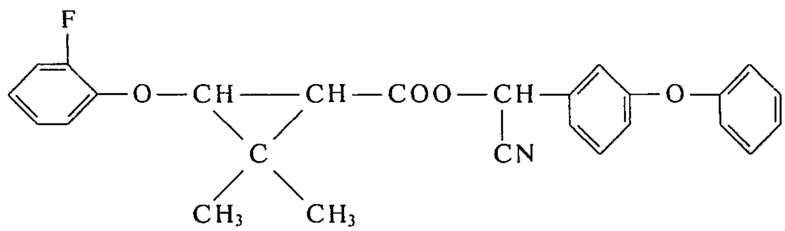

10. The compound according to claim 1 of the formula

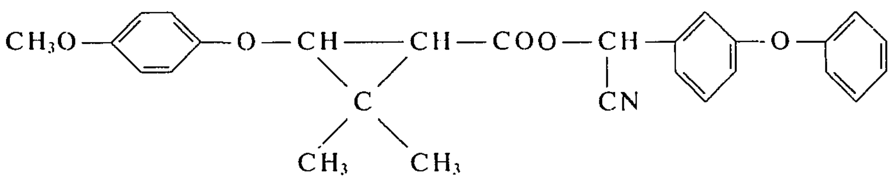

11. The compound according to claim 1 of the formula

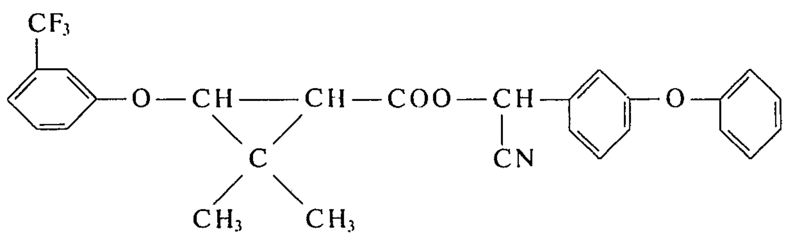

12. The compound according to claim 1 of the formula

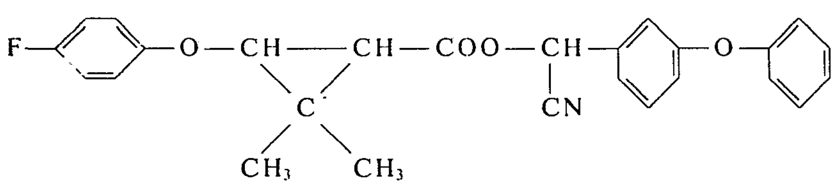

13. The compound according to claim 1 of the formula

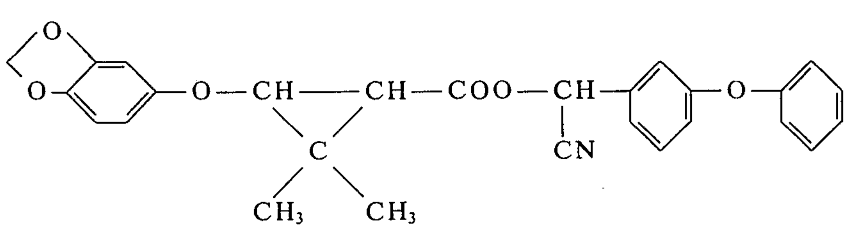

14. A process for the manufacture of a compound according to claim 1, characterised in that a compound of the formula

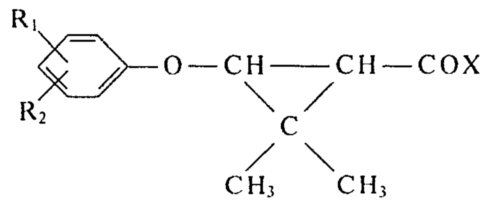

wherein $R_1$ and $R_2$ are as defined in claim 1 and X represents halogen, is reacted, in the presence of an acid acceptor, with the compound of the formula

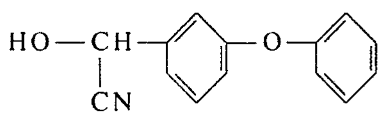

15. A process for the manufacture of a compound according to claim 1, characterised in that the compound of the formula VII

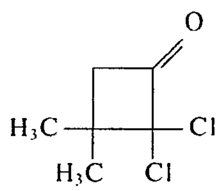

(VII)

is rearranged, in the presence of a catalyst, to give a compound of the formula (VIII)

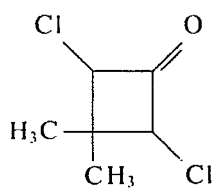

(VIII)

and the compounds (VII) or (VIII) are converted with phenols of the formula (IX)

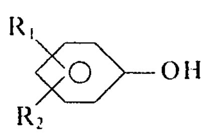

(IX)

wherein $R_1$ and $R_2$ are as defined in claim 1, into compounds of the formula (X)

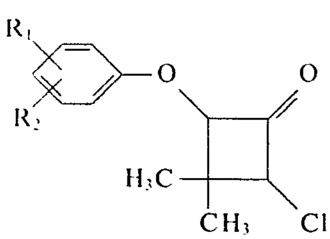

(X)

and subsequently ring contraction is effected with a base to give compounds of the formula (XI)

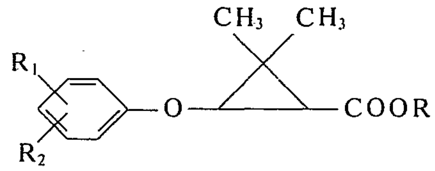

(XI)

wherein R is hydrogen or lower alkyl, and the compounds of the formula (XI) are reacted with the alcohol of the formula

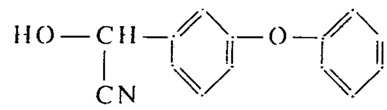

(V)

16. A pesticidal composition which contains a compound according to claim 1 as active component and a suitable carrier and/or other adjuvant.

17. A method of controlling a variety of animal and plant pests which comprises the use of a compound according to claim 1.

18. A method according to claim 17 wherein the pests to be controlled are insects and representatives of the order Acarina.

**Revendications**

1. Un ester d'acide cyclopropane-carboxylique de formule

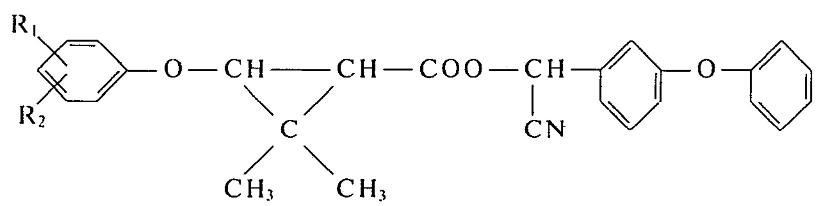

dans laquelle $R_1$ et $R_2$ représentent chacun l'hydrogène, un halogène, un groupe méthyle, méthoxy, trifluorométhyle ou cyano ou bien $R_1$ et $R_2$ représentent ensemble un groupe méthylène-dioxy ou le fragment $-CH=CH-CH=CH-$.

2. Le composé selon la revendication 1, de formule

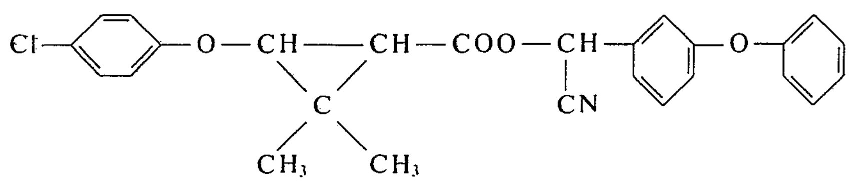

3. Le composé selon la revendication 1, de formule

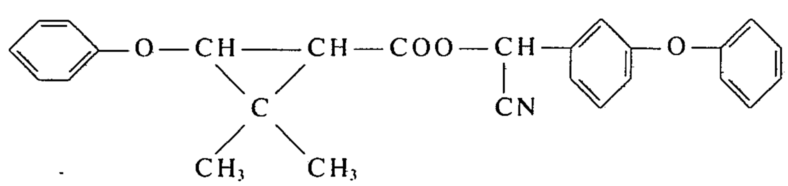

4. Le composé selon la revendication 1, de formule

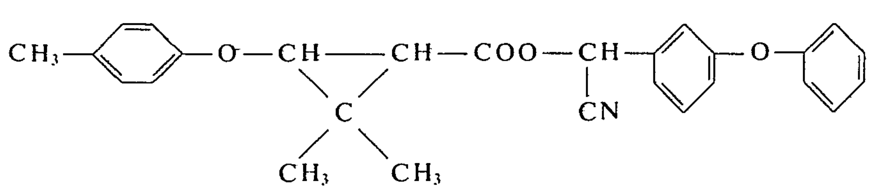

5. Le composé selon la revendication 1, de formule

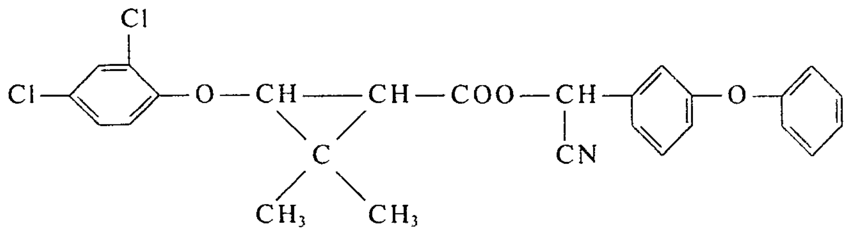

6. Le composé selon la revendication 1, de formule

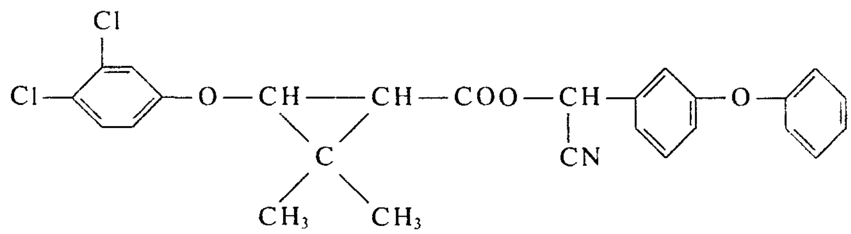

7. Le composé selon la revendication 1, de formule

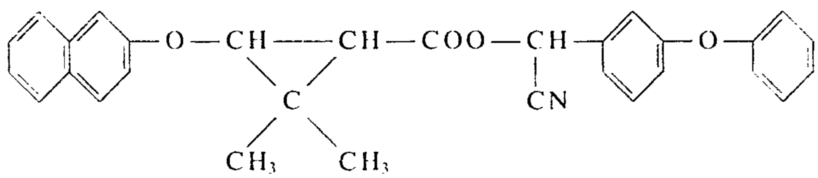

8. Le composé selon la revendication 1, de formule

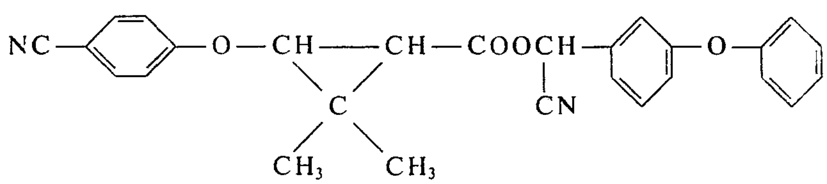

9. Le composé selon la revendication 1, de formule

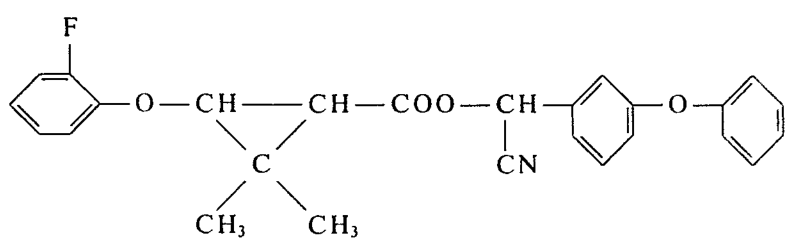

10. Le composé selon la revendication 1, de formule

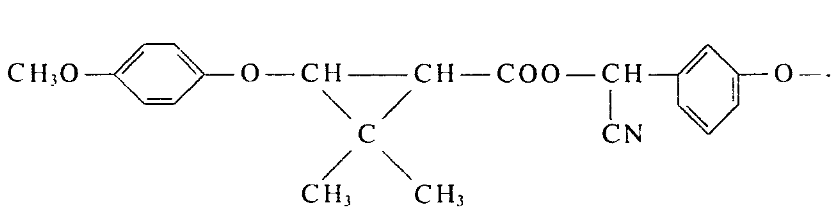

11. Le composé selon la revendication 1, de formule

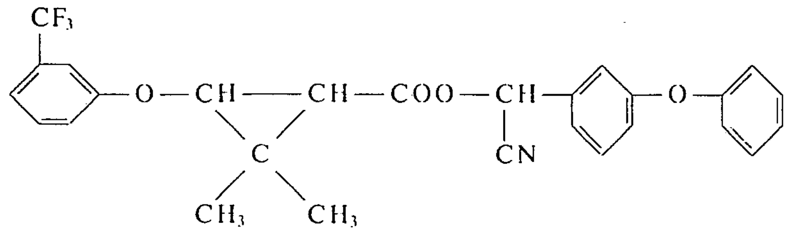

12. Le composé selon la revendication 1, de formule

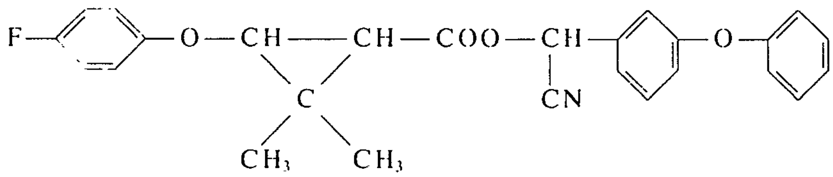

13. Le composé selon la revendication 1, de formule

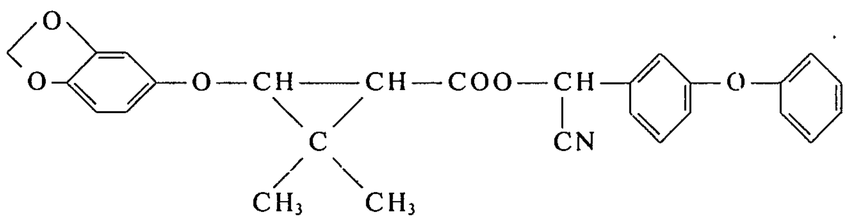

14. Procédé de préparation d'un composé selon l'une des revendications 1 à 13, caractérisé en ce que l'on fait réagir un composé de formule

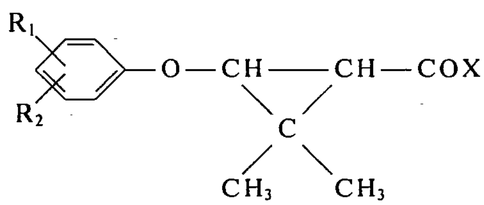

dans laquelle $R_1$ et $R_2$ ont les significations indiquées dans la revendication 1 et X représente un halogène, en présence d'un agent fixant les acides, avec le composé de formule

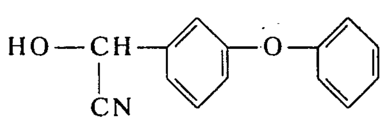

15. Procédé de préparation d'un composé selon la revendication 1, caractérisé en ce que l'on transpose le composé de formule VII

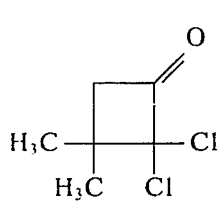

(VII)

en présence d'un catalyseur, en un composé de formule VIII

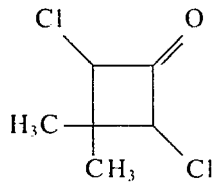

(VIII)

on convertit les composés VII ou VIII, par réaction avec des phénols de formule IX

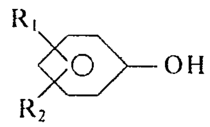

(IX)

dans laquelle $R_1$ et $R_2$ ont les significations indiquées dans la revendication 1, en composés de formule X

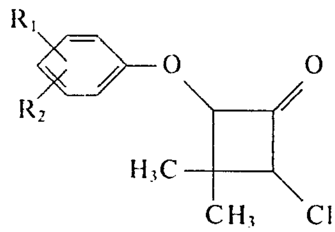

(X)

puis on provoque à l'aide d'une base le raccourcissement du cycle conduisant aux composés de formule XI

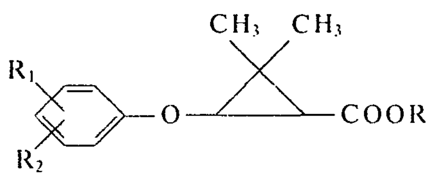

(XI)

dans laquelle R représente l'hydrogène ou un groupe alkyle inférieur et on fait réagir les composés de formule XI avec l'alcool de formule

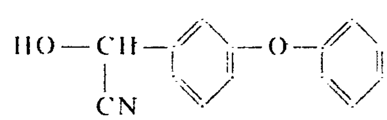

(V)

16. Un produit pesticide contenant en tant que composant actif un composé selon la revendication 1 et un véhicule et/ou un autre additif approprié.

17. Utilisation d'un composé selon la revendication 1 dans la lutte contre les parasites animaux et végétaux de types variés.

18. Utilisation selon la revendication 17, dans la lutte contre les insectes et les représentants de l'ordre des acariens.